# EUROPEAN PATENT SPECIFICATION

(11) **EP 1 955 646 B1**
(45) Date of publication and mention of the grant of the patent: **24.02.2010**
(21) Application number: 08009381.8
(22) Date of filing: 30.04.2004
(51) Int. Cl.: A61B 1/015

(54) **Endoscope fluid control input device**
Vorrichtung zur Endoskopfluidkontrolle
Dispositif de contrôle de fluide pour endoscope

(30) Priority: 12.05.2003 JP 2003133705
(43) Date of publication of application: 13.08.2008
(62) Divisional of application: 04730704.6
(73) Proprietor: Olympus Corporation, Tokyo 151-0072 (JP)
(72) Inventor: Arai, Keiichi, c/o Olympus Medical Systems Corporation, Tokyo 151-0072 (JP); Ikeda, Yuichi, c/o Olympus Medical Systems Corporation, Tokyo 151-0072 (JP); Miyagi, Takayasu, c/o Olympus Medical Systems Corporation, Tokyo 151-0072 (JP); Maeda, Toshinari, c/o Olympus Corporation, Tokyo 151-0072 (JP)
(74) Representative: von Hellfeld, Axel

(56) References cited:
- WO-A-98/02894
- GB-A- 2 335 080
- US-A- 5 872 527
- US-A- 5 938 589
- US-B1- 6 303 887

## Description

### Technical Field

The present invention relates to a fluid control input device for endoscope, and more particularly, to a fluid control input device for endoscope applied to a fluid control apparatus for endoscope, which supplies the fluid by using a channel arranged in an endoscope and absorbs the intracavital tissue, serving as an operating member upon controlling the supply and absorption of the fluid.

### Background Art

Recently, an endoscope has been widely used in the medical field. In the endoscope used for the medical field, when an endoscope inserting unit is inserted in the body cavity, the intracavital body fluid, dirt, and mucus are necessarily adhered to an observing window arranged to the distal end of the inserting unit, and the observation via the observing window is thus not sufficiently performed.

Therefore, usually, a conventional general endoscope has an air-supply and water-supply function, by which an operating button arranged to an operating unit on the hand side is operated to perform the solution supply operation and the observing window is thus cleaned or the air supply operation is performed to blow off the solution remaining on the surface of the observing window after the cleaning operation. The air supply function is also used for the case of intracavitally supplying the air so as to easily observe a desired observing portion.

Further, the conventional endoscope has an absorbing function, by which the tissue, such as the intracavital target part or diseased part, is partly absorbed and is obtained as specimen. The operation of air supply and water supply and the absorbing operation are performed via an air supplying channel, a water supplying channel, or an absorbing channel arranged in the endoscope.

Japanese Unexamined Patent Application Publication No. 2000-189380 and the like conventionally discloses and proposes various fluid control apparatuses for performing the water supply operation, the air supply operation, and the absorbing operation and operating members (fluid control input devices for endoscope (hereinafter, properly abbreviated to fluid control input devices)) arranged to the operating unit, for operating the fluid control apparatuses in the above-mentioned endoscopes.

In the operating member of the fluid control apparatus in the endoscope apparatus disclosed in Japanese Unexamined Patent Application Publication No. 2000-189380, a user presses-in an operating button, a cylinder which descends in accordance with the pressing operation is arranged to a switch main body, a shading plate is integrally arranged to the bottom of the cylinder, a photo-interrupter is arranged at the shading position in accordance with the descending operation of the shading plate, a reflecting plate is arranged to the bottom surface of the cylinder, predetermined beams are irradiated to the reflecting plate, and a reflecting-type photo-interrupter is arranged at the facing position of the reflecting plate so as to receive reflecting light of the reflecting plate. The output of the reflecting-type photo-interrupter is varied depending on the amount of pressing operation of the operating button.

In the operating member with the above-mentioned structure, the cylinder and the shading plate descend in accordance with the pressing operation of the operating button. Thus, the shading plate shades the reflecting-type photo-interrupter. Then, at the timing for shading the photo-interrupter by the shading plate, the absorbing operation (or air supply or water supply) operation starts. Corresponding to the output of the reflecting-type photo-interrupter in accordance with the amount of pressing operation of the operating button, the amount of absorption (or the amount of air supplied or the amount of water supplied) is controlled. Therefore, only the simple operation for pressing the operating button enables the fine adjustment of the amount of absorption (the amount of air supplied or the amount of water supplied).

In the operating member in the above form, a first-step operation of one operating member controls the on/off operation, and a second-step operation performs the fine adjustment of the amount of operation in the on-state as one example (referred to as a first example).

In another example of the operating member disclosed in Japanese Unexamined Patent Application Publication No. 2000-189380, a first-step operation with a predetermined amount of operation performs the air supply operation, the operation is switched to the water supply operation at a predetermined position, and a second-step and subsequent-step operation performs the water supply operation, as a two-step switch (referred to as a second example).

The operating member in the second example has a switch main body integrally having a cylinder, and the cylinder has, at the top end thereof, an air supply button which is repulsed in the pressing-up direction of a spring member. In the center of the switch main body, a water supply button is arranged, and the water supply button has a hole portion into which the air supply button is inputted upon pressing the air supply button. The water supply button is also repulsed by a spring member in the pressing-up direction to the switch main body.

On the bottom of the cylinder, a shading plate is integrally arranged. At the shading position in accordance with the descending operation of the shading plate, two photo-interrupters for air supply and water supply are laminated. On the bottom surface of the cylinder, a reflecting plate is arranged, predetermined beams are irradiated to the reflecting plate, and a reflecting-type photo-interrupter is arranged at the facing position of the reflecting plate so as to receive the reflecting light of the reflecting plate. The reflecting-type photo-interrupter detects the amount of light received, which changes depending on the amount of pressing operation of the air supply button, thereby controlling the water supply operation.

An example of an operating member of another two-step switch is disclosed as follows (referred to as a third example).

The operating member in the third example has the same structure as that of the operating member in the second example. However, the reflecting plate and the reflecting-type photo-interrupter are not used, the two photo-interrupters laminated in the second example are arranged in parallel, and, corresponding to this, a shading cylinder for shading a photo-interrupter for water supply and a photo-interrupter for air supply is integrally arranged to the cylinder. By setting the length of the shading plate to be varied, the corresponding photo-interrupter is shaded at the varying timing in accordance with the pressing operation of the operating button.

In the operating members in the second and third examples, the start operation and the fine adjustment of the air supply operation are performed in the first operation, and the air supply button comes into contact with the top surface of the water supply button. At the timing for starting to press the water supply button (second operation), the operation is switched from the air supply operation to the water supply operation. And, the water supply button is further pressed-in, thereby controlling the water supply operation. In this case, just before the first operation is switched to the second operation, the air supply operation is performed. At the position just before the switching operation, the amount of air supplied is set to be maximum in the air supply operation. In the second operation, that is, at the maximum pressing position of the water supply button, the amount of water supplied is set to be maximum in the water supply operation.

However, means disclosed in Japanese Unexamined Patent Application Publication No. 2000-189380 does not control a plurality of operations since the one operating member in the first example performs the start of one operation and the fine adjustment thereof.

In the operating members in the second and third examples, the first operation performs the start of the air supply operation and fine adjustment thereof, the air supply button comes into contact with the top surface of the water supply button and the water supply button is pressed. Then, the operation is switched from the air supply operation to the water supply operation, and the one operating member controls and switches the two operations.

However, the timing for switching the air supply operation corresponding to the first operation and the water supply operation corresponding to the second operation is set to a predetermined timing after the air supply button at the first step comes into contact with the water supply button at the second step. The switching timing is not precisely determined. Thus, an unnecessary amount of force is applied to the operating finger in the operation at the maximum position of the air supply operation at the first step and, then, the operation can be undesirably switched to the water supply operation. Therefore, in the case of the air supply operation to expand the stomach, an operator has to pay utmost attention to prevent the excessive pressing operation of the air supply button and simultaneously needs the operation for continuing the half-pressing operation of the operating member (operation for keeping a predetermined position within the range of the first operation). Further, upon erroneously switching to the water supply operation, the desired result in the observation using the endoscope is not obtained.

After the air supply button at the first step comes into contact with the water supply button at the second step, until actually switching operation, for some time, a so-called dead zone without the change in signal is set. Therefore, in accordance with the contact sense of the air supply button and the water supply button, the switching timing of the two operations is notified to the operator. However, the notifying means is not active and the switching timing is not easily recognized.

Further, in the operation for pressing the air supply button at the first step, a zone without generating the signal at the initial operation thereof, that is, the zone until the shading plate first shades the photo-interrupter becomes the so-called dead zone. With the above-mentioned setting, as the amount of pressing operation (operating stroke) of the entire operating member is increased, the dimension of the operating member in the height direction is increased. Thus, the size of the operating member is liable to be increased. When the size of the operating member is increased, an operator having relatively small hands (or relatively short fingers) cannot easily operate the operating member.

Meanwhile, the operating member disclosed in Japanese Unexamined Patent Application Publication No. 2000-189380 has a large number of components, such as springs. The internal mechanism of the operating member is complicated. Thus, when the dirt or stain enters the operating member, the operating member is not cleaned easily.

Then, in order to prevent the intrusion of the dirt or stain, a movable portion of the air supply button and the water supply button has a waterproof seal and the like. However, at the portion having the waterproof seal, the component is always slid in the operation. Therefore, the portion is used for a long time and then the portion is worn and the waterproof ability gradually deteriorates.

The present invention is devised in consideration of the circumstances. It is an object of the present invention to provide a fluid control input device for endoscope, in which the operator easily and certainly recognizes the switching point of two operations or the timing after/before the switching operation with the dust-proof and waterproof structure, even upon using the operating member having the two-step switch.

Document US 5,938,589 discloses an air/water feed switch portion disposed in an endoscope control section configured so that a push button, comprising a first piston body and a second piston body is moved vertically by using a spring member in an upper and lower support cylinders. The second piston body, which turns on/off a water feed switch is operated with an operation stroke which is longer than the stroke of the first piston body, which turns on/off the air feed switch.

### Disclosure of Invention

It is an object of the present invention to provide a fluid control input device allowing different modes of operation.

A fluid control input device in accordance with the present invention is described in claim 1. Preferred embodiments of that device are described in the dependent claims.

### Brief Description of the Drawings

Fig. 1 is a schematic diagram showing the schematic structure of fluid channels and pipes in a fluid control apparatus connected to an endoscope used for a fluid control input device according to a first embodiment of the present invention;
Fig. 2 is a sectional view showing the detailed structure of the fluid control input device shown in Fig. 1;
Fig. 3 is a diagram showing a change relationship between the amount of pressing force and the amount of air supplied upon pressing the fluid control input device shown in Fig. 1 and the change in amount of pressing force upon switching from the air supply operation to the water supply operation;
Fig. 4 is a sectional view showing a main portion of the structure of a cutout of the components in a fluid control input device according to a modification of the first embodiment of the present invention;
Fig. 5 is an enlarged sectional view showing a main portion of an enlarged periphery of a click mechanism in the fluid control input device shown in Fig. 4 according to the modification;
Fig. 6 is a sectional view showing the detailed structure of a fluid control input device according to a second embodiment of the present invention;
Fig. 7 is a sectional view showing the structure of a fluid control input device according to a third embodiment of the present invention;
Fig. 8 is a schematic diagram showing the structure of a fluid control input device according to one modification of the third embodiment of the present invention;
Fig. 9 is a sectional view showing the structure of a fluid control input device according to a fourth embodiment of the present invention; and
Fig. 10 is a sectional view showing the structure of a fluid control input device according to a fifth embodiment of the present invention.

### Best Mode for Carrying Out the Invention

Hereinbelow, embodiments of the present invention will be described with reference to the drawings.

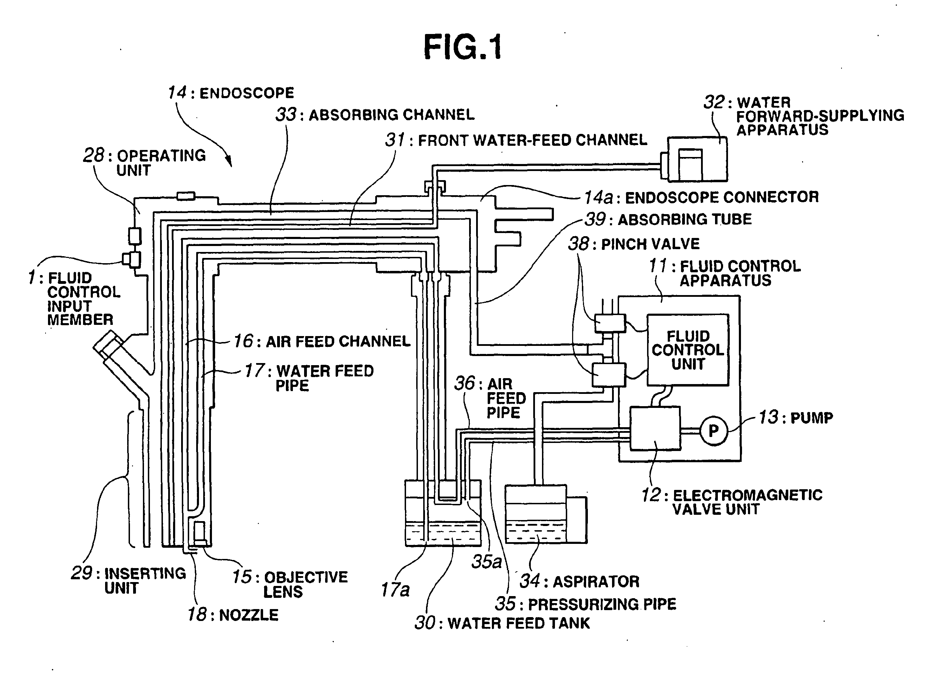

Fig. 1 is a schematic diagram showing the schematic structure of fluid channels and pipes in a fluid control apparatus connected to an endoscope used for a fluid control input device according to the first embodiment of the present invention.

Referring to Fig. 1, an endoscope 14 comprises: an operating unit 28; and an inserting unit 29. In the endoscope 14, an air supplying channel 16, a water supplying channel 17, a water forward-supplying channel 31, and an absorbing channel 33 are formed. The operating unit 28 comprises various operating members, e.g., a fluid control input member 1, serving as a fluid control input device. The inserting unit 29 has, at the distal end thereof, image pickup means (not shown), and has, on the front surface of the distal end thereof, an objective lens 15 and openings of the channels.

The air supplying channel 16 and the water supplying channel 17 are formed to merge to one channel near the distal end of the inserting unit 29. Near the opening of the front surface of the one channel, a nozzle 18 is arranged. The nozzle 18 is shaped to spray a predetermined fluid toward the surface of the objective lens 15, and is arranged at a predetermined position near the objective lens 15.

The water forward-supplying channel 31 is inserted in the inserting unit 29 and the operating unit 28 of the endoscope 14. Further, the water forward-supplying channel 31 is extended from an endoscope connector 14a and thereafter is connected to a water forward-supplying device 32.

Similarly, the water supplying channel 17 is inserted in the inserting unit 29 and the operating unit 28 of the endoscope 14. The water supplying channel 17 is extended from the endoscope connector 14a, and thereafter is connected to a water supply tank 30 for supplying the water to the water supplying channel 17. In this case, a distal opening 17a of the water supplying channel 17 is arranged at a predetermined position to be always sunk in the water pooled in the water supply tank 30.

Similarly, the air supplying channel 16 is inserted in the inserting unit 29 and the operating unit 28 of the endoscope 14. The air supplying channel 16 is extended from the endoscope connector 14a and thereafter is guided to the water supply tank 30. Then, the air supplying channel 16 is connected to an air supplying pipe 36 via the water supply tank 30.

The air supplying pipe 36 is connected to an electromagnetic valve unit 12 arranged in a fluid control apparatus 11. From the electromagnetic valve unit 12, a pressurizing pipe 35 for pressurizing the inside of the water supply tank 30 is connected to the water supply tank 30. In this case, a distal opening 35a of the pressurizing pipe 35 is arranged at a predetermined position where it is opened in the water supply tank 30 and it is not sunk in the water pooled in the water supply tank 30. Incidentally, a pump 13 drives the electromagnetic valve unit 12 of the fluid control apparatus 11.

Incidentally, the electromagnetic valve unit 12 comprises a plurality of electromagnetic valves (individual electromagnetic valve is not shown), e.g., electromagnetic valves corresponding to the use for water supply operation and air supply operation. In accordance therewith, the pump 13 comprises a plurality of pumps (individual pump is not shown), e.g., pumps corresponding to the use for water supply operation and air supply operation. The individual pump drives the corresponding electromagnetic valve.

Further, the absorbing channel 33 is similarly inserted in the inserting unit 29 and the operating unit 28 of the endoscope 14. The absorbing channel 33 is extended from the endoscope connector 14a and thereafter is guided to an aspirator 34 via an absorbing valve 39 and a leak tube inserted to two pinch valves 38 controlled by the fluid control apparatus 11.

Meanwhile, as mentioned above, the operating members are arranged to the operating unit 28 of the endoscope 14. Among the operating members, a predetermined signal line (not shown) for transmitting a signal generated by the fluid control input member 1 (e.g., an output signal of an optical sensor 2, which will be described later) is connected to the fluid control apparatus 11 via the inside of the endoscope 14 and the endoscope connector 14a.

Therefore, the signal outputted from the fluid control input member 1 is transmitted to the electromagnetic valve unit 12 of the fluid control apparatus 11. Thus, among the plurality of electromagnetic valves (not shown) of the electromagnetic valve unit 12, the corresponding electromagnetic value is opened/closed. In accordance therewith, various operations are performed, e.g., the air supply operation starts or ends, the amount of air supplied increases or decreases, the water supply operation starts or ends, and the spray operation starts or ends.

A signal from another predetermined operating member (absorbing operating member) different from the fluid control input member 1 is transmitted from the fluid control apparatus 11 to the two pinch valves 38. Then, in response thereto, the pinch valves 38 opens/closes the absorbing valve 39 and the leak tube and the absorbing operation starts or ends, and the amount of absorption increases/decreases.

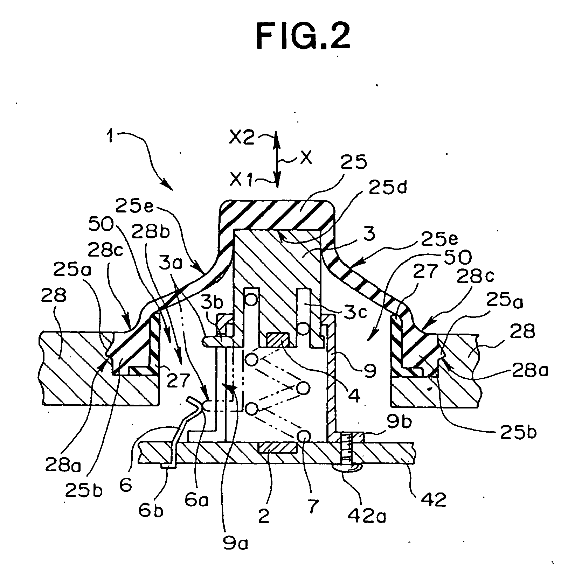

Fig. 2 is a sectional view showing the detailed structure of the fluid control input device (fluid control input member 1) according to the first embodiment.

Referring to Fig. 2, the fluid control input member 1 according to the first embodiment comprises: a rubber cover 25; an operating button 3; a button receiving member 9; a fixing substrate 42; a repulsive spring 7; a click spring 6; a light-emitting device 4; and the optical sensor 2.

The rubber cover 25 is watertight covering means for waterproof operation or a covering member. The rubber cover 25 watertightly covers the entire components, such as the operating button 3, serving as moving means or a moving member, partly forming the fluid control input member 1 and is bent in a predetermined form in accordance with the pressing operation of the operating button 3.

The operating button 3 is moving means that is movably arranged in the direction shown by an arrow X1 in Fig. 2.

The button receiving member 9 supports the operating button 3, and guides the moving direction of the operating button 3.

The button receiving member 9 is fixed to the fixing substrate 42.

The repulsive spring 7 is arranged in the inner space of the button receiving member 9 to be sandwiched between the operating button 3 and the fixing substrate 42, and is position restoring means or a restoring member, containing a coil spring or the like, which repulses the operating button 3 in the direction shown by an arrow X2 shown in Fig. 2.

The click spring 6 partly forms a click mechanism, and is arranged, indicating the switching point of a first-step switch and a second-step switch so as to form the fluid control input member 1 as a two-step switch. As will be described later, the click spring 6 is amount-of-force changing means or an amount-of-force changing mechanism, which generates temporary and sharp change in amount of force.

The light-emitting device 4 is arranged to the bottom surface of the operating button 3, and irradiates predetermined luminous fluxes.

The optical sensor 2 is arranged at a predetermined position facing the light-emitting device 4 on the surface of the fixing substrate 42, and is signal generating means or a detecting member of the amount of operation which receives luminous fluxes from the light-emitting device 4, converts the luminous fluxes into a predetermined electrical signal, and outputs the converted signal.

Preferably, the rubber cover 25 contains a silicon rubber or fluorocarbon rubber with the higher chemical-resistance and repeating bending resistance.

The fixing substrate 42 is a fixing member, such as an electric substrate, and is arranged at a predetermined position in the operating unit 28 (refer to Fig. 1) of the endoscope 14. At a predetermined position of the fixing substrate 42, the button receiving member 9 is fixed by using a fixing member 42a, such as a screw.

As mentioned above, on the surface of the fixing substrate 42, the optical sensor 2 is arranged with the light receiving surface thereof directed to the top. The positioning of the operating button 3 and the button receiving member 9 is set to arrange the irradiating surface of the light-emitting device 4 at the position facing the light receiving surface of the optical sensor 2. That is, the optical sensor 2 is arranged at a predetermined position within the irradiating range of the luminous fluxes from the light-emitting device 4. The positions of the optical sensor 2 and the light-emitting device 4 are set for the optical sensor 2 to receive the luminous fluxes outputted from the light-emitting device 4.

As mentioned above, the repulsive spring 7 constantly repulses the operating button 3 in the direction shown by the arrow X2 shown in Fig. 2. Thus, the resistance of the pressing operation with a predetermined amount of force acts to the operating button 3. An operator presses the operating button 3 in the direction shown by an arrow X1 shown in Fig. 2. Thereafter, the amount of pressing force is reset and then the operating button 3 is repulsed and moved in the direction shown by the arrow X2 shown in Fig. 2. The operating button 3 is finally restored to a predetermined position.

The operating button 3 is a substantially cylindrical member having a flange unit 3b at the bottom end thereof, and has projected portions 3a, serving as engaging portions, which are projected to the outer circumference from the flange unit 3b at three portions with the same interval.

The button receiving member 9 is a substantially cylindrical member having a flange unit 9b at the bottom end, with openings at both ends thereof. A screw hole in which the fixing member 42a is screwed is pierced through the flange unit 9b. Thus, the button receiving member 9 is fixed with a screw to the fixing substrate 42. Three groove notches 9a are formed onto the outer circumference of the button receiving member 9 with the same interval. The groove notches 9a are formed onto the outer circumference of the button receiving member 9, at the periphery ranging from the bottom end to the top end, and are opened to the bottom end and are closed to the top end.

Therefore, the operating button 3 is inserted in the button receiving member 9. Then, the projected portions 3a of the operating button 3 are slidably engaged with the groove notches 9a. Thus, the operating button 3 is moved in the direction shown by an arrow X without rotation. The operating button 3 fit from the bottom side of the button receiving member 9 is not pulled out to the side of the top end. That is, the groove notches 9a have a function of guiding means of the operating button 3, and further have a positioning function at the top end of the operating button 3.

On the other hand, when the operating button 3 is fit into the button receiving member 9, the repulsive spring 7 is arranged in the button receiving member 9. In this case, on the side of the bottom end of the operating button 3, a circular groove portion 3c for fitting the top end of the repulsive spring 7 like a coil is pierced. Therefore, the repulsive spring 7 is positioned in the button receiving member 9. In this state, the button receiving member 9 is fixed to the fixing substrate 42, thereby operating the repulsive force of the repulsive spring 7 in the extending direction from the bottom end of the operating button 3. The operating button 3 is always repulsed in the direction shown by the arrow X2 shown in Fig. 2.

When the projected portions 3a of the operating button 3 are fit into the groove notches 9a of the button receiving member 9, the projected portions 3a are set to be slightly projected to the outside from the outer circumference of the button receiving member 9.

At positions corresponding to the three projected portions 3a, the click springs 6 which are formed by bending a plate spring or the like are arranged. The click springs 6 have fixing ends 6b at one end thereof which are fixed at predetermined positions of the fixing substrate 42. Further, the click springs 6 have free ends 6a at the other end thereof which are slightly projected on the locus for moving the projected portions 3a.

Therefore, the operating button 3 is moved in the X direction and then the projected portions 3a come into contact with the free ends 6a of the click springs 6 at predetermined positions (refer to reference numeral 3a at the position shown by a dotted line in Fig. 2). In this case, the projected portions 3a of the operating button 3 press the click springs 6 against the repulsive force of the click springs 6 and are moved in the direction shown by the arrow X. When the projected portions 3a go over the click spring 6, the predetermined sense of click operation is generated. The sense of click operation is generated just before switching the first-step operation and the second-step operation of the fluid control input member 1. As mentioned above, the click spring 6 and the projected portions 3a form the click mechanism, serving as amount-of-force changing means or an amount-of-force changing mechanism, which generates the temporary and sharp change in amount of force.

The optical sensor 2 receives the luminous fluxes from the light-emitting device 4, converts the received luminous fluxes into a predetermined electrical signal, and thereafter outputs the electrical signal. In this case, the output signal of the optical sensor 2 changes, depending on the strength of luminous fluxes received. In accordance with the movement of the operating button 3 in the direction shown by the arrow X, the light-emitting device 4 is moved in the same moving direction. Therefore, the strength of luminous fluxes received by the optical sensor 2 changes, depending on a separate distance between the light-emitting device 4 and the optical sensor 2.

Therefore, the output signal of the optical sensor 2 changes, depending on the change in the position corresponding to the amount of pressing force of the operating button 3 having the light-emitting device 4. Thus, the light-emitting device 4 and the optical sensor 2 have, as one set, a function of position detecting means which detects the position or movement of the operating button 3.

As mentioned above, the output signal of the optical sensor 2 is transmitted to the fluid control apparatus 11 via a predetermined signal line. Therefore, the fluid control apparatus 11 receives the signal outputted from the optical sensor 2, and detects the position or the amount of movement of the operating button 3 based on the strength in received signal. The fluid control apparatus 11 controls the pump 13 and the electromagnetic valve unit 12 based on the detecting result to control the operation of a plurality of electromagnetic valves, thereby controlling the switching of a fluid (air and water) supplied to the endoscope 14 or the amount of fluid flow.

The operating button 3 is arranged to be externally projected from a hole portion 28b pierced at a predetermined position of the operating unit 28 in the endoscope 14. The rubber cover 25 containing an elastic modifying member is arranged so as to cover the hole portion 28b.

In this case, a ring rubber insert 27 having the L-shaped cross-section is adhered and fixed to the inner circumference of the hole portion 28b of the operating unit 28. A groove portion 28c is formed to the inner circumference of the hole portion 28b of the operating unit 28. An end 25b of the rubber cover 25 is fit and adhered to the groove portion 28c.

A circumferential groove 28a is formed to the inner wall surface of the hole portion 28b. Corresponding to this, a projected portion 25a circumferentially-formed is formed to the outer circumference of the end 25b of the rubber cover 25. When the rubber cover 25 is fit to the groove portion 28c of the operating unit 28, the circumferential groove 28a of the operating unit 28 is fit into the projected portion 25a with the tightening degree for flatten the projected portion 25a of the rubber cover 25. Thus, a dust-proof and waterproof structure is obtained in the hole portion 28b of the operating unit 28. The entire components of the fluid control input member 1 are watertightly covered with the rubber cover 25.

An inside surface 25d of the top of the rubber cover 25 is adhered and fixed to the top surface of the operating button 3. The rubber cover 25 has a smooth curved portion at the portion facing the portion (shown by reference numeral 25e) ranging from the top surface of the operating button 3 to the side surface. Further, as mentioned above, the end 25b of the rubber cover 25 is fit and adhered to a predetermined position (groove portion 28c) of the operating unit 28.

Thus, in accordance with the pressing operation of the operating button 3, the curved portion 25e is folded into a space 50 between the button receiving member 9 and the hole portion 28b and then the rubber cover 25 is bent in a predetermined form. In the process of the bending, the temporary and sharp change in amount of force is generated. The timing for generating the temporary and sharp change in amount of force of the rubber cover 25 is set at a predetermined timing after/before the generating timing corresponding to the generating timing of a predetermined signal outputted from the optical sensor 2 in accordance with the positional change in the operating button 3. The timing for generating the change in amount of force is set to the same timing for generating the signal, and the timing for generating the change in amount of force can be arbitrarily set.

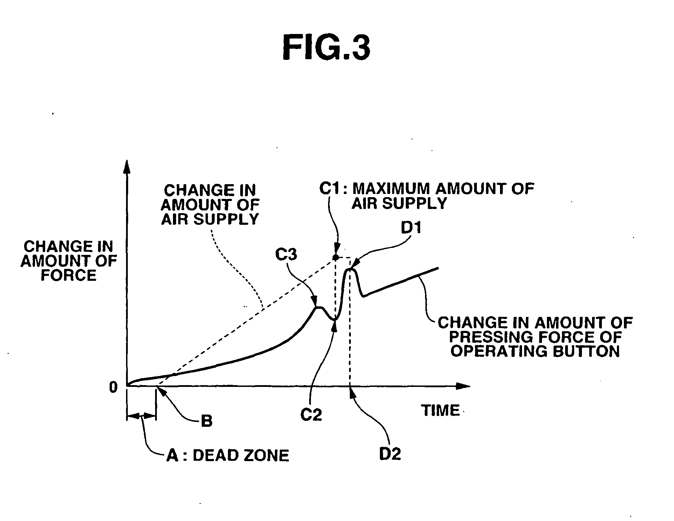

The operation upon operating the fluid control input member 1 with the above-mentioned structure is as follows. Incidentally, Fig. 3 is a diagram showing a change relationship between the amount of pressing force and the amount of air supplied upon pressing the operating button 3 and the change in amount of pressing force upon switching from the air supply operation to the water supply operation.

First, the operator presses the top surface of the operating button 3 from the rubber cover 25 in the direction shown by the arrow X1, thereby moving the operating button 3 in the same direction. In this case, the light emission of the light-emitting device 4 starts. The light emission starts upon starting a main power supply of the endoscope system using the endoscope 14. Alternatively, in response to the start of movement of the operating button 3, the light emission may start. In this case, predetermined means for detecting the movement of the operating button 3 is provided.

In response to the start of light emission of the light-emitting device 4, the optical sensor 2 operates and then the optical sensor 2 receives the luminous fluxes from the light-emitting device 4 and outputs a predetermined signal (information indicating that the operating button 3 operates and a signal indicating positional information on the operating button 3) to the fluid control apparatus 11. In response to this, the fluid control apparatus 11 controls the pump 13 and the electromagnetic valve unit 12, thereby driving a predetermined electromagnetic valve (electromagnetic valve for air supply, not shown). Then, the air supply operation for starting to supply the air in the body cavity having the endoscope 14 via the air supplying pipe 36 and the air supplying channel 16 starts (refer to reference numeral B shown Fig. 3).

Therefore, a predetermined time lag exists during the period from starting the pressing operation of the operating button 3 to actually starting the air supply operation. The time lag is a dead zone shown by reference numeral A in Fig. 3. Incidentally, the time of the dead zone A is arbitrarily set. For example, when the time of the dead zone A is set to zero, even if the fluid control input member 1 is touched at the undesired timing, the air supply operation promptly starts. In order to prevent such a situation, preferably, a predetermined time of the dead zone A is set. When the dead zone A is long, the amount of pressing operation (operating stroke) of the operating button 3 increases. Then, the size of the fluid control input member 1 increases and therefore the dead zone A needs to be properly set in consideration of this.

In accordance with the amount of pressing operation of the operating button 3, the output signal of the optical sensor 2 changes. In accordance therewith, the fluid control apparatus 11 controls the operation for increasing the output of a pump for air supply in the pump 13. Further, in accordance with this, the degree of opening of the corresponding electromagnetic valve for air supply increases, and the amount of air supplied increases as shown by a dotted line in Fig. 3.

Here, upon pressing the operating button 3 in the same direction, the restoring force of the repulsive spring 7 gradually increases in accordance with the amount of pressing operation. The curvature of the smooth curved portion (25e) formed to the rubber cover 25 gradually reduces. In accordance with this, the elastic restoring force of the rubber cover 25 is generated. Therefore, the amount of pressing force of the operating button 3 needs to be increased (refer to Fig. 3).

In accordance with the movement of the operating button 3, the light-emitting device 4 is moved in the same direction. The space between the light-emitting device 4 and the optical sensor 2 gets narrow. Thus, the strength of luminous fluxes received by the optical sensor 2 increases. Therefore, as mentioned above, the output signal of the optical sensor 2 changes depending on the amount of pressing operation of the operating button 3.

Thus, upon pressing the operating button 3, at the timing at which the amount of air supplied is maximum (refer to reference numeral C1 in Fig. 3), the bending of the rubber cover 25 is maximum and thereafter a predetermined portion (curved portion 25e) of the rubber cover 25 is folded in the direction (approximately parallel direction) along the moving direction (direction shown by the arrow X) of the operating button 3 toward the hole portion 28b. That is, in this state, the predetermined portion (curved portion 25e) of the rubber cover 25 is accommodated in a predetermined space of the hole portion 28b (refer to reference numeral C2 in Fig. 3).

In the state just before the above state, that is, just before the state in which the predetermined portion (curved portion 25e) of the rubber cover 25 is folded, the amount of force obtained by adding the amount of elastic restoring force of the rubber cover 25 to the amount of restoring force of the repulsive spring 7 (both the force is repulsive force in the direction shown by the arrow X2) operates, as the resistant sense against the finger of the operator which presses the operating button 3 (refer to reference numeral C3). Thereafter, at the timing at which the predetermined portion (curved portion 25e) of the rubber cover 25 is bent and is folded, the amount of force in the direction shown by the arrow X1 temporarily and sharply operates (refer to reference numeral C2).

Therefore, at the moment, the resistant sense against the finger of the operator temporarily and sharply reduces, thereby generating so-called sense of click operation. Thus, the operator clearly recognizes the fact. That is, the operator recognizes that the rubber cover 25 is bent and is folded, thereby recognizing that the amount of air supplied is set to be maximum.

In this state, the pressing operation of the operating button 3 stops and the state keeps. Then, the air supply operation continues while keeping the maximum amount of air supplied. During this, the operator performs the intracavital observation, examination, and treatment.

In the halfway of the above operation, when the dirt and the like is adhered to the surface of the objective lens 15 arranged to the endoscope 14, the operator performs the water supply operation, thereby cleaning the surface of the objective lens 15.

In order to clean the objective lens 15, the operating button 3 is further pressed from the state. Then, the projected portions 3a of the flange unit 3b of the operating button 3 come into contact with the click springs 6 (refer to the position 3a shown by the dotted line in Fig. 2). Further, the operating button 3 is pressed and then the repulsive force of the click spring 6 operates to the operating button 3. A large amount of pressing force of the operating button 3 is required. When the projected portions 3a go over the click springs 6 (refer to reference numeral D1 in Fig. 3), the amount of pressing force sharply reduces. Thus, the operator obtains the sense of click operation.

In response to the output of the optical sensor 2 just after the timing (reference numeral D), the fluid control apparatus 11 performs the switching control processing from the air supply operation to the water supply operation (refer to reference numeral D2 in Fig. 3). That is, the fluid control apparatus 11 controls the pump 13 and the electromagnetic valve unit 12, thereby driving a predetermined electromagnetic valve (electromagnetic valve for water supply, not shown). Then, the air supply operation to the pressurizing pipe 35 pressurizes the inside of the water supply tank 30, thereby starting the water supply operation for supplying the water (solution) in the water supply tank 30 to the body cavity having the endoscope 14 via the water supplying channel 17.

Therefore, the operator presses the operating button 3 with the amount of force against the repulsive force of the click springs 6, and obtains the sense of click operation when the operating button 3 goes over the click springs 6. After that, the operator recognizes the start of water supply operation.

In other words, the fluid control input device 1 according to the first embodiment is notifying means which sends, to the operator, a notification indicating that the switching operation from the air supply operation to the water supply operation is performed by generating a predetermined signal from the optical sensor 2, by using the change in amount of force (the sense of click operation) generated by the amount-of-force changing means (amount-of-force changing mechanism) (click mechanism) including the click springs 6 and the projected portions 3a.

In the state in which the operation switched to the water supply operation, the surface of the objective lens 15 is cleaned or the like.

After executing the water supply operation and cleaning the surface of the objective lens 15, the amount of pressing force of the operating button 3 is reduced, the restoring force obtained by adding the elastic force of the repulsive spring 7 and the elastic restoring force of the rubber cover 25 operates to the operating button 3, and then the operating button 3 is pressed-up in the direction shown by the arrow X2 shown in Fig. 2. Thus, the amount of water supplied is gradually reduced. In response to the output signal from the optical sensor 2 at a predetermined timing (reference numeral D2) just before the projected portions 3a go over the click springs 6, the fluid control apparatus 11 is switched from the fluid operation to the air supply operation, thereby performing predetermined control processing for spraying the remaining water in the merged channel of the air supplying channel and the water supplying channel on the back side of a nozzle.

Further, the amount of pressing force of the operating button 3 is reduced, thereby gradually reducing the amount of air supplied. The folding state of the rubber cover 25 is restored to the original state shown in Fig. 2. Even in this state, the elastic force of the repulsive spring 7 in the direction shown by the arrow X2 operates to the operating button 3, thereby continuously moving the operating button 3 in the same direction. The operating button 3 is pressed and returned and is positioned at a predetermined point until the flange unit 3b comes into contact with an end of the groove notch 9a of the button receiving member 9 (refer to reference numeral 3b shown by a solid line in Fig. 2). At the timing just before the above state (refer to reference numeral B), the air supply operation ends.

As mentioned above, according to the first embodiment, the sense of click operation generated by the operation when the rubber cover 25 is bent and is folded in accordance with the pressing-down operation of the operating button 3 and the sense of click operation generated when the projected portions 3a go over the click springs 6 are clearly transmitted to the finger of the operator. At a predetermined timing after the sense of click operation is generated, the operation is switched from the air supply operation to the water supply operation.

That is, at a predetermined timing in the processing for pressing-down the operating button 3, the fluid control apparatus 11 switches the operation from the air supply operation to the water supply operation. The operator certainly recognizes the switching timing.

Therefore, although the two-step switch executes the two operations including the air supply operation and the water supply operation by using one operating member and the two-step switch forms the fluid control input member 1, the desired operation is executed without switching the two operations by the erroneous operation and the preferable operability is ensured.

Since the user clearly recognizes the timing at which the amount of air supplied is maximum, upon continuing the air supply operation, the amount of pressing operation of the operating button 3, that is, the target of the amount of air supplied is easily estimated. Therefore, the preferable operability is ensured.

Further, the two-step switch structure with the simple structure is realized and this contributes to the reduction in the number of parts and in manufacturing costs. The switching operation of the first step and the second step of the two-step switch electrically detects the position in the noncontact state by using the light-emitting device 4 and the optical sensor 2. Therefore, the structure is highly durable, as compared with the structure using an electrical switch having a conventional electrical contact or the like.

Since the so-called dead zone does not need to be set before/after the switching timing of the operation at the first step (air supply operation) and the operation at the second step (water supply operation), the entire pressing process (distance stroke) of the operating button 3 is reduced and therefore the dimension of the operating member in the height direction is suppressed. Therefore, this contributes to the reduction in size of the endoscope using the fluid control input member 1.

In the conventional two-step switch, the amount of pressing force in the first step and the amount of folding force in the second step change. Thus, a repulsive spring contributing to the operation in the first step and a repulsive spring contributing to the operation in the second step are individually arranged. Further, means for changing the amounts of repulsive force of both the repulsive springs can be provided by using the repulsive spring in the second step containing a wire with a thick diameter or using two repulsive members with different spring constants. However, in this case, the repulsive member with the large amount of repulsive force results in the deterioration in operability. Then, according to the first embodiment, the repulsive spring 7 may be one member which can be operated with the same amount of force in the first step and the operation in the second step. Hence, the preferable operability is easily ensured, and the simple structure of components contributes to the reduction in manufacturing costs.

In the general case, an elastic member, such as a rubber cover, is arranged to the portion which the operator directly touches, such as the operating member. Then, as a buffer material against the amount of force transmitted to the finger of the operator, the rubber cover operates. Therefore, it is well-known that the operator cannot recognize the slight change in amount of force of the repulsive spring generated at the switching point or contact sense. However, in the fluid control input member 1 according to the first embodiment, in the processing for pressing-down the rubber cover 25 together with the operating button 3, the rubber cover 25 is bent. Therefore, the operator clearly recognizes the switching point of the operation.

Further, in the fluid control input member 1 according to the first embodiment, the rubber cover 25 entirely covers the components in the fluid control input member 1. Thus, the amount of pressing force of the operating button 3 is reduced and the dust-proof and waterproof structure is formed by using the rubber cover 25. An operating portion of the operating button 3 does not need the waterproof seal. This contributes to the reduction in components and further to the reduction in manufacturing costs. Simultaneously, the seal member is not provided and there is no danger of the wear of seal member. The high resistance is ensured and the repeating use is possible.

Incidentally, the fluid control input member 1 according to the first embodiment uses the optical sensor 2 and the light-emitting device 4, as position detecting means that detects the position of the operating button 3. However, the present invention is not limited to this position detecting means. For example, another position detecting means including a magnetic member and a magnetic sensor may be used.

When the rubber cover 25 is bent and is folded, the sense of click operation is generated. Then, just after the sense of click operation is generated when the projected portions 3a of the operating button 3 go over the click springs 6, the operation is switched from the air supply operation to the water supply operation. The switching timing is not limited to this. For example, the operation may be switched simultaneously with the timing at which the projected portions 3a go over the click springs 6.

Incidentally, according to the first embodiment, in accordance with the output signal from the optical sensor 2 at the timing just after the sense of click operation is generated when the projected portions 3a go over the click springs 6, the fluid control apparatus 11 performs predetermined control processing for switching the operation from the fluid operation to the air supply operation. In addition, the click spring 6 may be used as an operation change-over switch. In this case, the click springs 6 are moved by the projected portions 3a, thereby generating a predetermined signal. The signal is transmitted to the fluid control apparatus 11. In response to the signal, the fluid control apparatus 11 performs predetermined operation switching control processing. Thus, the same advantages as those according to the first embodiment are obtained.

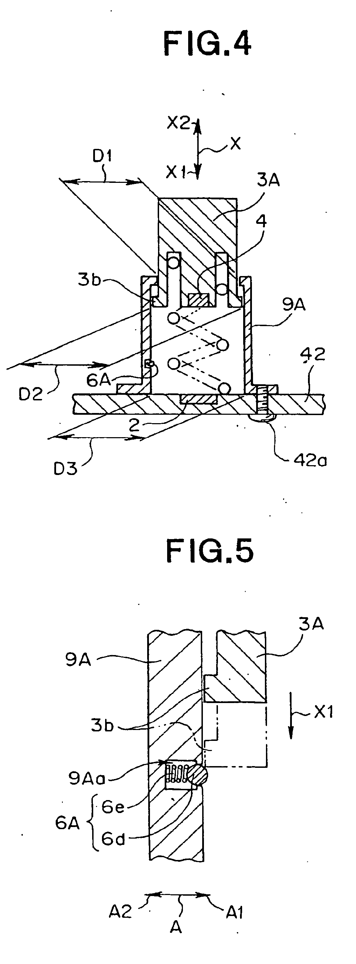

According to the first embodiment, as mentioned above, the switching point of the first step and the second step forming the two-step switch structure using the click spring 6 is shown. However, the present invention is not limited to the above structure. For example, the structure shown in Fig. 4 may be used and the same structure can be obtained.

Fig. 4 is a sectional view showing a main portion of the structure of partly cutoff components in a fluid control apparatus according to one modification of the first embodiment of the present invention.

The structure of a fluid control input member (1), serving as a fluid control input device according to the modification is basically the same as that according to the first embodiment. Only the structure of a click mechanism forming the two-step switch structure is different from that according to the first embodiment. Therefore, referring to Fig. 4, only different portions are described and the remaining portions are not described in detail with reference to the description using Fig. 2.

Referring to Fig. 4, the flange unit 3b, serving as an engaging portion of an operating button 3A of the fluid control input member (1), according to the modification has a smaller amount of projection, as compared with that of the flange unit 3b of the operating button 3 in the fluid control input member 1 according to the first embodiment. In this case, a diameter D2 of the outermost-circumference of the flange unit 3b is pierced through the top surface of the button receiving member 9, and is set so that it is slightly larger than a diameter D1 of a hole portion on the inner circumference, to which the operating button 3A is projected and is substantially equal to or slightly smaller than a diameter D3 (diameter of the hole portion pierced through the bottom surface of the button receiving member 9) of the inner-circumferential wall surface of the button receiving member 9.

The operating button 3 is inserted in the button receiving member 9A and the button receiving member 9A supports the movement of the operating button 3 in the direction shown by an arrow X. The button receiving member 9A is formed by omitting the groove notch 9a according to the first embodiment. That is, the button receiving member 9 has hole portions with diameters D1 and D3 and predetermined shapes on the top surface and the bottom surface thereof. On the side of the lower end (side of the fixing substrate 42) of the button receiving member 9, the button receiving member 9A has the flange unit 9b for fixing itself to the fixing substrate 42. Thus, the button receiving member 9A is fixed and supported to the top surface of the fixing substrate 42 by using the same means (fixing member 42a, such as a screw) as that according to the first embodiment.

In place of the click springs 6 forming the click mechanism according to the first embodiment, at a predetermined position of the inner wall surface of the button receiving member 9A, a click member 6A is arranged, including a ball plunger including a ball 6d and a coil spring 6e.

Fig. 5 is an enlarged sectional view showing a main portion of an enlarged periphery of a click mechanism in the fluid control input device shown in Fig. 4 according to the modification.

Referring to Fig. 5, the click member 6A forming the click mechanism comprises the ball 6d and the coil spring 6e for repulsing the ball 6d. The ball 6d and the coil spring 6e are arranged in hole portions 9Aa arranged at predetermined plural positions of the inner wall surface of the button receiving member 9A. That is, the coil spring 6e is fixed to the bottom surface of the portion 9Aa, and the coil spring 6e repulses the ball 6d in the direction shown by an arrow A1 in Fig. 5. In this case, the diameter of the opening of the portion 9Aa is slightly smaller than the diameter of the ball 6d. Therefore, the fall of the ball 6d from the portion 9Aa to the outside due to the coil spring 6e is prevented. The ball 6d repulsed in the direction shown by the arrow A1 by the coil spring 6e is partly projected to the outside from the opening with a predetermined amount.

In this state, the operator presses the operating button 3A, thereby moving the flange unit 3b of the operating button 3A in the direction shown by the arrow X1 in Fig. 5. Then, the operating button 3A comes into contact with the ball 6d as shown by a dotted line in Fig. 5.

Further, the operating button 3A is pressed and then the ball 6d is moved against the repulsive force of coil spring 6e in the direction of the inside of the hole portion 9Aa, that is, in the direction shown by an arrow A2 in Fig. 5. In this case, the repulsive force of the coil spring 6e operates to the flange unit 3b of the operating button 3A. Thus, the amount of pressing force of the operating button 3A needs to be slightly increased.

The flange unit 3b of the operating button 3A passes through the position facing the hole portion 9Aa (the ball 6d). The repulsive force of the coil spring 6e returns the ball 6d to the original position. Then, the repulsive force of the coil spring 6e to the operating button 3A is sharply released. Therefore, the operator of the operating button 3A obtains predetermined sense of click operation.

When the operator releases the amount of pressing force to the operating button 3A to return the operating button 3A to the original position, similarly the sense of click operation is obtained.

As mentioned above, according to the modification, the same advantages as those according to the first embodiment are obtained.

Next, a description is given of a fluid control input device according to a second embodiment of the present invention.

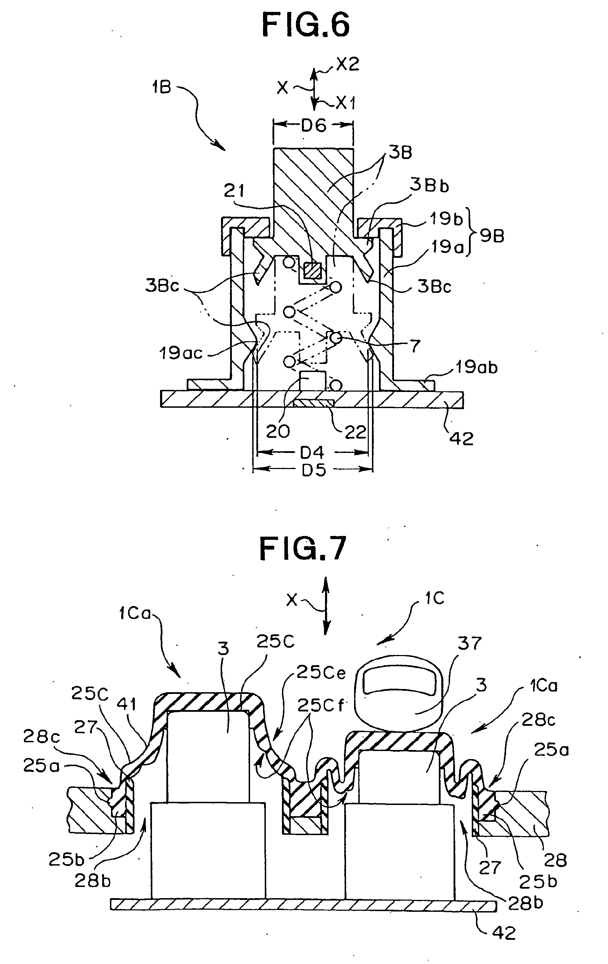

Fig. 6 is a sectional view showing the detailed structure of a fluid control input device (fluid control input member 1B) according to the second embodiment of the present invention.

The structure according to the second embodiment basically uses the structure according to the first embodiment. However, the structures of the operating button, the button receiving member for receiving the operating button, and the click mechanism are different from those according to the first embodiment. Therefore, the same components as those according to the first embodiment are designated by the same reference numerals and a detailed description thereof is omitted. According to the second embodiment, the rubber cover for entirely covering the fluid control input device is arranged. The rubber cover is arranged similarly to that according to the first embodiment, and the drawing is not shown for the purpose of simple drawing. An endoscope using the fluid control input device according to the second embodiment and a fluid control apparatus connected to the endoscope are the same as those according to the first embodiment, therefore, are referred to Fig. 1, and a drawing and a description thereof are not shown.

Referring to Fig. 6, the fluid control input member 1B, serving as a fluid control input device, according to the second embodiment comprises: a waterproof rubber cover (not shown, refer to Fig. 2); the operating button 3B that is arranged movably in the direction shown by an arrow X in Fig. 6; the button receiving member 9B that supports the operating button 3 and guides the moving direction; the fixing substrate 42 to which the button receiving member 9B is fixed; the coil-shaped repulsive spring 7 that is arranged in the inner space of the button receiving member 9B and repulses the operating button 3B in the direction shown by the arrow X2 in Fig. 6; a magnetic member 21, such as a magnet, which is arranged near the bottom end of the operating button 3B; a magnetic sensor 22, serving as signal generating means, which is arranged at a predetermined position facing the operating button 3B on the surface of the fixing substrate 42; and a micro switch 20.

The button receiving member 9B has the structure including two members of a supporting portion 19a and a flange cover 19b. The supporting portion 19a is a substantially cylindrical member having openings at both end surfaces thereof. On one end surface of the supporting portion 19a, the flange unit 19ab is formed. Via the flange unit 19ab, the button receiving member 9B is fixed onto the surface of the fixing substrate 42. As means for fixing the button receiving member 9B to the fixing substrate 42, the means according to the first embodiment is used. At a predetermined position of the inner side-wall surface of the supporting portion 19a in the button receiving member 9B, a click projected portion 19ac, serving as an engaged portion, circumferentially-formed throughout the entire periphery is formed.

The operating button 3B is a substantially cylindrical member having the opening at one end. At the one end having the opening, a flange unit 3Bb is formed. At the bottom portion of the flange unit 3Bb, a click spring foot 3Bc, constituting with six engaging portions with the elasticity, is formed. Incidentally, the click spring foot 3Bc contains, e.g., thermoplastic resin (polypropylene), and is integrally formed to the operating button 3B.

The click spring foot 3Bc has a distal end which is wide to the outside so that it is wider than an outer-diameter dimension D6 of the operating button 3. An outer-diameter dimension D5 at the distal end is set to be slightly larger than an outer-diameter dimension D4 (refer to Fig. 6) at the top portion of the click projected portion 19ac of the button receiving member 9B. Therefore, upon moving the operating button 3B in the direction shown by an arrow X in Fig. 6, the click spring foot 3Bc comes into contact with the click projected portion 19ac of the button receiving member 9B and goes over the click projected portion 19ac, thereby forming the click mechanism for generating the sense of click operation.

The micro switch 20 is arranged at a predetermined position with which a predetermined portion of the distal end of the click spring foot 3Bc of the operating button 3B comes into contact. Thus, upon pressing the operating button 3B in the direction shown by the arrow X1 at the highest level, a switch portion of the micro switch 20 is pressed and the resultant generated signal is transmitted to the fluid control apparatus 11 (refer to Fig. 1).

Incidentally, the magnetic member 21 is integrally arranged to the operating button 3B, the distance between the magnetic member 21 and the magnetic sensor 22 changes in accordance with the movement of the operating button 3B in the direction shown by the arrow X. Therefore, the magnetic sensor 22 detects the level of the magnetic field of the magnetic member 21, thereby detecting the distance between the magnetic sensor 22 and the magnetic member 21, that is, positional information of the operating button 3B. The detecting result obtained by the magnetic sensor 22 is transmitted to the fluid control apparatus 11.

The operation of the fluid control input member 1B with the above-mentioned structure is as follows.

That is, the operator presses the operating button 3B, thereby moving the operating button 3B in the direction shown by an arrow X1 shown in Fig. 6. In accordance with the pressing operation of the operating button 3B, the bending of a rubber cover (not shown) starts. Then, the click spring foot 3Bc comes into contact with the click projected portion 19ac of the button receiving member 9B. Thereafter, the click spring foot 3Bc is modified toward the inside and goes over the click projected portions 19ac (in the state shown by a dotted line in Fig. 6).

Just before the timing at which the click spring foot 3Bc goes over the click projected portion 19ac, a rubber cover (not shown) is bent and is folded similarly to the case according to the first embodiment. Thus, the sense of click operation is generated.

Before the click spring foot 3Bc comes into contact with the click projected portions 19ac, the amount of pressing force of the operating button 3B is gradually increased by the operation when the rubber cover is bent. At the moment at which the rubber cover is folded, the amount of pressing force of the operating button 3B sharply reduces.

Thereafter, while the click spring foot 3Bc comes into contact with the click projected portion 19ac and goes over them, the elastic force of the click spring foot 3Bc gradually increases the amount of pressing force of the operating button 3B. When the click spring foot 3Bc goes over the click projected portion 19ac, the amount of pressing force of the operating button 3B sharply reduces at the timing. Thus, the sense of click operation is generated.

At the just-after timing, a predetermined portion of the operating button 3B presses the switch portion of the micro switch 20, thereby generating a predetermined signal from the micro switch 20. The generated signal is transmitted to the fluid control apparatus 11. In response to the transmitted signal, the fluid control apparatus 11 executes the switching control processing from the air supply operation to the water supply operation.

That is, according to the second embodiment, the pressing operation of the operating button 3B starts the air supply operation. In accordance with the pressing operation of the operating button 3B, the amount of air supplied increases. The amount of air supplied is increased at the timing before/after generating the sense of click operation when the rubber cover is folded at the timing before the click spring foot 3Bc comes into contact with the click projected portion 19ac.

Just after generating the sense of click operation when the click spring foot 3Bc goes over the click projected portion 19ac, the micro switch 20 operates, thereby switching the operation from the air supply operation to the water supply operation.

As mentioned above, according to the second embodiment, the same advantages as those according to the first embodiment are obtained.

Since a switching signal from the air supply operation to the water supply operation is generated by using the micro switch 20, the control for switching the operation is more certainly executed and this contributes to the reduction in parts.

According to the second embodiment, when the operating button 3B is moved in the direction shown by the arrow X1, the amount of air supplied is set to be maximum at the timing for generating the sense of click operation (first time) by using the rubber cover. The air supply operation is switched to the water supply operation at the timing for generating the sense of click operation (second time) by using the click mechanism (having the click spring foot 3Bc and the click projected portion 19ac). The operation starting at the timing for generating the sense of click operation is not limited to this and may be executed by combining other operations.

According to the second embodiment, the click projected portion 19ac is formed to the button receiving member 9B at only one position. In addition, for example, the similar-shaped projected portion is arranged so that the click spring foot 3Bc goes over the two click projected portions (19ac) two times upon moving the operating button 3B. Then, the sense of click operation is generated three times, including the sense of click operation when the rubber cover is folded upon moving the operating button 3B in the direction shown by the arrow X1 or in the direction shown by the arrow X2.

In this case, at the first timing for generating the sense of click operation (using the rubber cover), the amount of air supplied is set to be maximum. At the second timing of generating the sense of click operation (at the first time with the click mechanism), the spray operation starts. At the third timing for generating the sense of click operation (at the second time with the click mechanism), the operation is switched to the water supply operation.

In the case of starting the operation or switching the operation at the timing for generating the sense of click operation, the setting of the so-called dead zone is not necessary. Therefore, the entire pressing stoke of the operating button 3 is reduced. The dimension of the operating member in the height direction is suppressed and this contributes to the reduction in size in device.

In the case of arranging the two click projected portions (19ac) as mentioned above, the dimensions of the click projected portions 19ac in the height direction are different. Then, it is possible to increase/reduce the sense of click operation which is generated by the corresponding click projected portions 19ac. Therefore, the type of the previous operation is always recognized by the sense of click operation, and the next operation is easily grasped only by obtaining the operating sense.

Next, a description is given of a fluid control input device according to the third embodiment of the present invention.

Fig. 7 is a sectional view showing the structure of a fluid control input device (fluid control input member 1C) according to a third embodiment of the present invention.

According to the third embodiment, two two-step switches are used. Therefore, the structure of each of the switches (fluid control input device) basically uses the structure according to the first embodiment. However, the structure of a rubber cover 25C is slightly different from that according to the first embodiment. Therefore, the same components according to the first embodiment are designated by the same reference numerals and a detailed description thereof is omitted. An endoscope using the fluid control input device according to the third embodiment and a fluid control apparatus connected to the endoscope are the same as those according to the first embodiment and therefore, Fig. 1 is referred to Fig. 1, and the drawing and description thereof are omitted.

Referring to Fig. 7, the fluid control input member 1C according to the third embodiment comprises: the two two-step switches; and the waterproof rubber cover 25C for covering the entire two two-step switches 1Ca with a single member.

Similarly to the case according to the first embodiment, one of the two two-step switches 1Ca has a function of an air-supply and water-supply switch which performs the air supply operation and the water supply operation. The other two-step switch has a function of an absorbing switch.

The two two-step switches 1Ca have components except for the rubber cover 25C, which are the same as those in the fluid control input member 1 according to the first embodiment. The rubber cover 25C has substantially the same structure as that according to the first embodiment, and comprises the two rubber covers 25 according to the first embodiment to cover the entire two two-step switches 1Ca.

That is, at the outer-circumference of the end 25b of the rubber cover 25C, the circumferentially formed projected portion 25a is formed. Corresponding to this, the circumferential groove 28a is formed to the inner wall surface of the hole portion 28b of the operating unit 28 (also refer to Fig. 1) having the two two-step switches 1Ca. The ring rubber insert 27 is adhered and fixed to the inner-circumference of the hole portion 28b, thereby forming the groove portion 28c. The end 25b of the rubber cover 25C is fit and adhered to the groove portion 28c.

Therefore, when the rubber cover 25C is fit into the groove portion 28c of the operating unit 28, the projected portion 25a of the rubber cover 25C is fit into the circumferential groove 28a of the operating unit 28. Thus, the dust-proof and waterproof structure of the hole portion 28b is obtained.

At predetermined portion of the rubber cover 25C, that is, at predetermined part of the inside of a curved portion 25Ce, a slightly thinner portion than other portions is formed (thin portion 25Cf). The operating button 3 is pressed to the thin portion 25Cf. In accordance therewith, when the rubber cover 25C is bent and is folded, the rubber cover 25C is folded with the thin portion 25Cf, as the top. That is, the thin portion 25Cf is a folding portion. By arranging the thin portion 25Cf, the rubber cover 25C is bent without fail and is always folded in the same form.

Incidentally, according to the third embodiment, one of the two two-step switches 1Ca, as an air-supply and water-supply switch, has the same operation as that according to the first embodiment.

The other absorbing switch is set to have the maximum amount of absorption when the operating button 3 is further pressed to the position of the maximum stroke farther than the position for generating the sense of click operation by the rubber cover 25C. The operation of the absorbing switch is the same as that according to the first embodiment.

That is, as the operating button 3 is pressed in the direction shown by the arrow X1, the curvature of the curved portion 25Ce in the rubber cover 25C gradually reduces. In accordance therewith, the reactive force generated by the rubber cover 25C increases. Thus, the amount of pressing force of the operating button 3 increases.

Further, the operating button 3 is pressed and then the curvature of the curved portion 25Ce of the rubber cover 25C cannot be kept. The curved portion 25Ce of the rubber cover 25C is bent to the inside, and is accommodated in a predetermined space of the hole portion 28b (refer to Fig. 7). Reference numeral 37 in Fig. 7 shows the finger of the operator. Therefore, referring to Fig. 7, the operating button 3 of one of the two two-step switches 1Ca is pressed by a finger 37, and the rubber cover 25C corresponding to one of the two-step switches 1Ca is folded, thereby being accommodated in a predetermined space of the hole portion 28b.

In this case, the rubber cover 25C is folded with the thin portion 25Cf of the curved portion 25Ce, as the top. At the folding timing of the rubber cover 25C as mentioned above, the reactive force of the elastic restoring force of the rubber cover 25C is lost. Thus, the amount of pressing force of the operating button 3 is instantaneously reduced, thereby generating the sense of click operation. Hence, the operator recognizes that the operating button 3 is pressed to a predetermined portion.

According to the third embodiment, as described above, the same advantages as those according to the first embodiment are obtained. Further, the two two-step switches 1Ca are entirely covered with one rubber cover 25C with watertightness. The inner components of the two two-step switches 1Ca are always clean without the dirt adhering to the inner components of the two two-step switches 1Ca, that is, operating portions such as a spring and a switch.

Since the space between the two two-step switches 1Ca is covered with the rubber cover 25C, the periphery of the fluid control input member 1C easily cleaned.

For example, with manual operation for absorbing the tissue of the target part in a hood attached to the distal end of the endoscope and incising the tissue by squeezing the tissue with a high-frequency energizing wire, the amount of absorption must be controlled to be small. In this case, the operator easily adjusts the amount of pressing force within the range for preventing the generation of the sense of click operation using the rubber cover 25C. This contributes to the improvement in operability.

In the fluid control input member 1C having the aligned two two-step switches 1Ca, the rubber cover 25 for entirely and simultaneously covering the two two-step switches 1Ca is arranged, the thin portion 25Cf is arranged in the curved portion 25Ce of the rubber cover 25 so that the amount of pressing force bends to modify the shape of the rubber cover 25C toward the inside, and the folded space 50 is arranged. Hence, the modified portions of the rubber cover 25 for covering the two two-step switches 1Ca do not interfere with each other. Therefore, even in the case of simultaneously pressing the two two-step switches 1Ca, a predetermined corresponding portion of the rubber cover 25C with the thin portion 25Cf, as the top, is bent without fail and is folded to a predetermined position. This contributes to the improvement in operability.

Further, since the curved portion 25Ce of the rubber cover 25C is formed with the curved surface in the general state, the cleaning is easy and the rubber cover 25C is always clean.

Incidentally, the two two-step switches 1Ca according to the third embodiment have the same structure as that according to the first embodiment. However, the present invention is not limited to this. According to the third embodiment, the thin portion 25Cf is arranged at a predetermined portion in the curved portion 25Ce of the rubber cover 25C. Advantageously, the sense of click operation is clear when the rubber cover 25C is bent and is folded.

Then, the click mechanism (not shown) can be omitted from the two two-step switches 1Ca according to the third embodiment and the sense of click operation generated upon pressing the operating button 3 can be caused only by the rubber cover 25C. In this case, since the sense of click operation caused by using the rubber cover 25C is further clear, the same advantages as those according to the first embodiment are obtained.

As mentioned above according to the embodiments, based on the sense of click operation caused by the click mechanism on the side of the switch member or the sense of click operation generated when the rubber cover is pressed and is bent and is folded, the timing for starting or ending predetermined operations or the timing for adjusting the increase/decrease in amount of air supplied or the amount of water supplied is set in the present invention.

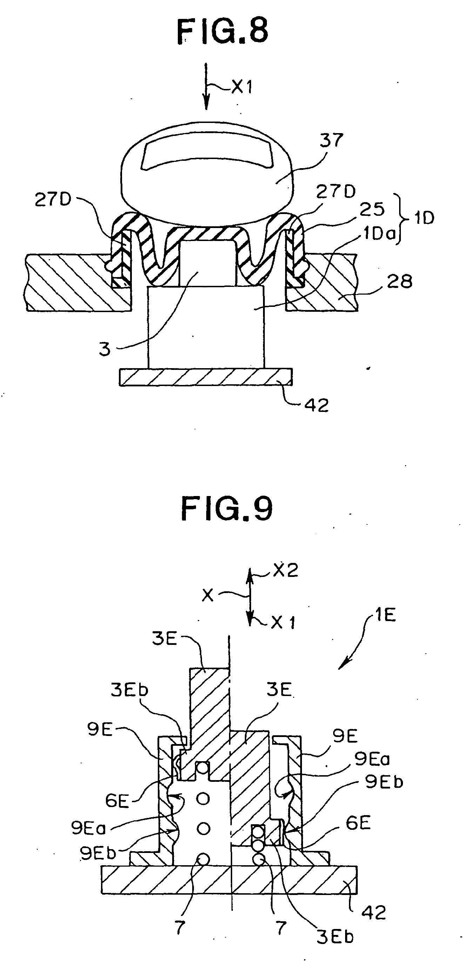

Then, in addition to the sense of click operation recognized by the operator, the sense of touch recognized by the finger of the operator can be used. Fig. 8 is a schematic diagram showing the structure of a fluid control input device (fluid control input member 1D), in which the operator recognizes the operating timing by using the sense of touch of the finger of the operator as well as the sense of click operation according to a modification of the third embodiment of the present invention.

The structure according to the modification of the third embodiment basically uses the structure according to the first embodiment. However, the structure of a ring rubber insert 27D arranged to fix the rubber cover 25 is slightly different from that according to the first embodiment. Therefore, the same components as those according to the first embodiment are not described in detail and are designated by the same reference numerals.

Referring to Fig. 8, the ring rubber insert 27D according to the modification of third embodiment has the longer dimension in the height direction, as compared with the dimension of the ring rubber insert 27 (refer to Fig. 2) according to the first embodiment.

In the fluid control input member 1D with the above-mentioned structure, the operating button 3 is pressed in the direction shown by an arrow X1 in Fig. 8 and then, similarly to the embodiments, the rubber cover 25 is bent and is folded. Thus, the sense of click operation is generated. Until this state, the air supply operation is performed.

The cushion of the finger 37 of the operator presses the operating button 3. In this case, the cushion of the finger 37 comes into contact with the center of the operating button 3 via the rubber cover 25. In this state, the operating button 3 is further pressed and then the circumference of the cushion of the finger 37 comes into contact with the position corresponding to the distal end of the ring rubber insert 27D, serving as the top of the folded rubber cover 25. In this case, the maximum amount of air supplied is set.

That is, when the cushion of the finger 37 of the operator and the circumference thereof come into contact with predetermined positions of the rubber cover 25 and the contact area between the rubber cover 25 and the cushion of the finger 37 of the operator increases, the amount of air supplied is set to be maximum. Therefore, the operator keeps the state, thereby keeping the maximum amount of air supplied. The operation is extremely easy.

From the state, the operating button 3 is further pressed with a predetermined amount of pressing force and the switching control processing is then performed from the air supply operation to the water supply operation. Predetermined position detecting means detects whether or not the operating button 3 is at a predetermined position, thereby executing the switching control processing. Incidentally, the position detecting means is, for example, an optical device or optical sensor, or a magnetic member or magnetic sensor.

According to the modification, as mentioned above, the same advantages as those according to the first embodiment are obtained. In addition, when the contact area between the cushion of the finger 37 for pressing the operating button 3 and the surface of the rubber cover 25 increases, that is, when the rubber cover 25 comes into contact with a predetermined portion (circumference) of the cushion of the finger 37, the maximum amount of fluid is set. Thus, the operator easily recognizes, by using the sense of touch of the rubber cover 25 which comes into contact with the finger 37, that the amount of fluid is set to be maximum. By adjusting the amount of pressing force of the operating button 3 to keep the sense of touch, the set maximum amount of fluid is easily kept. Further, from the state, only the pressing operation of the operating button 3 switches the type of fluid. The force of the finger is weakened so as to reduce the sense of touch, thereby performing the slight adjustment. Therefore, the erroneous operation is suppressed and this contributes to the improvement in operability.

Next, a description is given of a fluid control input device according to a fourth embodiment of the present invention.

Fig. 9 is a sectional view showing the structure of a fluid control input device (fluid control input member 1E), showing the normal state before operating the operating button at the left half portion and the pressing state of the operating button at the right half portion, according to the fourth embodiment of the present invention.

The structure according to the fourth embodiment basically uses the structure according to the first embodiment. However, the operating button and the button receiving member for receiving the operating button and the click mechanism thereof are different from the structure according to the first embodiment. Therefore, the same components as those according to the first embodiment are designated by the same reference numerals and a detailed description thereof is omitted. Further, according to the fourth embodiment, a rubber cover for entirely covering the fluid control input device is arranged. The rubber cover according to the fourth embodiment is arranged similarly to that according to the first embodiment and is not shown for the purpose of a brief description of the drawing. An endoscope using the fluid control input device according to the fourth embodiment and a fluid control apparatus connected to the endoscope are the same as those according to the first embodiment, therefore, are referred to Fig. 1, and a drawing and a description thereof are omitted. Only the different portions are described hereinbelow.

Similarly to the first embodiment, the fluid control input member 1E according to the fourth embodiment comprises: a rubber cover (not shown); an operating button 3E; and a button receiving member 9E. The button receiving member 9E is fixed and supported by predetermined fixing means (not shown) at a predetermined position of the top surface of the fixing substrate 42.

A flange unit 3Eb is formed on the side of the bottom end of the operating button 3. Click springs 6E, serving as a plurality of (e.g., three) engaging portions, are integrally fixed and supported to be projected toward the outside at predetermined positions on the outer-circumference of the flange unit 3Eb.

The button receiving member 9E comprises, on the inner wall surface thereof, a click caved portion 9Ea (caved portion) and a click projected portion 9Eb (projected portion), serving as engaged portions, corresponding to the click springs 6E. The click caved portion 9Ea and the click projected portion 9Eb, serving as the engaged portions may be caved with the groove along the inner wall surface and be projected continuously arranged. Or, the click caved portion 9Ea and the click projected portion 9Eb may be formed only at predetermined portions corresponding to the moving range of the click springs 6E.

With the above-mentioned structure, in the movement of the operating button 3E in the direction shown by an arrow X in Fig. 9, the click springs 6E pass through the click caved portion 9Ea and then are fit into the click caved portion 9Ea, thereby instantaneously reducing the amount of pressing force of the operating button 3. Thereafter, the amount of pressing force of the operating button 3 increases, thereby pulling-out the click springs 6E from the click caved portion 9Ea. Thus, the sense of click operation is obtained.

When the click springs 6E pass through the click projected portion 9Eb, the click springs 6E are pressed to the click projected portion 9Eb, thereby gradually increasing the amount of pressing force of the operating button 3. Then, the click springs 6E go over the click projected portion 9Eb, thereby instantaneously reducing the amount of pressing force of the operating button 3. Thus, the sense of click operation is obtained.

Incidentally, referring to Fig. 9, the position detecting means for detecting the position or the amount of movement of the operating button 3E is the same as that according to the first embodiment and a drawing thereof is omitted. Other structures are the same as those according to the first embodiment.

The operation of the fluid control input member 1E according to the fourth embodiment with the above structure is as follows. That is, in the state at the left half portion in Fig. 9, the operating button 3E is pressed in the direction shown by an arrow X1 in Fig. 9. Then, the operating button 3E is moved in the same direction. Simultaneously, predetermined operation of the air supply operation, or the like, starts.

Then, the click springs 6E integrally-fixed at predetermined positions of the operating button 3E are moved along the inner wall surface of the button receiving member 9E and are then fit into the click caved portion 9Ea. In accordance with the movement of the operating button 3E, the operation is controlled to gradually increase the mount of fluid due to the air supply operation, or the like.

When the click springs 6E are fit into the click caved portion 9Ea, the amount of pressing force of the operating button 3E is instantaneously reduced. Further, the operating button 3E is pressed and the click springs 6E are then pulled-out from the click caved portion 9Ea. The amount of pressing force in this case slightly increases.

When the click springs 6E are fit into the click caved portion 9Ea, the operating button 3E is temporarily positioned. Incidentally, the elasticity of the repulsive spring 7 in the direction shown by the arrow X2 (extending direction) in this case operates to the operating button 3E. The elasticity is set to the amount of force for preventing the pull-out of the click springs 6 from the click caved portion 9Ea. Therefore, if the pressing force is released from the operating button 3E in this state, the state is kept.

Subsequently, the operating button 3E is further pressed in the direction shown by the arrow X1. Then, the click springs 6E are detached from the click caved portion 9Ea and are moved in the same direction. Here, the switching control operation may be executed from the air supply operation to the water supply operation. Subsequently, the operating button 3 is pressed in the same direction, the water supply operation then continues, and the amount of fluid gradually increases.

Then, the click springs 6E come into contact with the click projected portion 9Eb. From this state, the operating button 3E is further pressed and the amount of pressing force gradually increases. When the click springs 6E are arranged to the top of the click projected portion 9Eb, the necessary amount of pressing force is maximum. Further, the operating button 3 is pressed and the click springs 6E go then over the top of the operating button 3. Instantaneously, the amount of pressing force of the operating button 3 reduces. The state is shown at the right half portion in Fig. 9. In this state, similarly to the above case (just after the sense of click operation at the first time), in the click springs 6E, the click projected portion 9Eb regulates the elasticity due to the repulsive spring 7 in the direction shown by the arrow X2, thereby stopping the operating button 3E at the position.

As mentioned above, in the fluid control input member 1E, the sense of click operation two times is obtained within the moving range of the operating button 3E in the direction shown by the arrow X. At a predetermined timing before/after generating the sense of click operation two times, the control operation is performed to adjust the amount of fluid by the air supply operation or end the air supply operation and to start or end the water supply operation and adjust the amount of fluid by the water supply operation. In addition, when the rubber cover 25 is bent and is folded, the sense of click operation can be generated. Thus, the operability is ensured with more certainty and the erroneous operation is easily suppressed.

Incidentally, the positional relationship between the click caved portion 9Ea and the click projected portion 9Eb is not limited to this. For example, referring to Fig. 9, the click projected portion 9Eb may be formed on the top side and the click caved portion 9Ea may be formed on the bottom. Further, in place of the click projected portion 9Eb shown in Fig. 9, a concaved portion similar to the click caved portion 9Ea may be formed, thereby combining the concaved portions. On the other hand, in place of the click caved portion 9Ea shown in Fig. 9, a projected portion similar to the click projected portion 9Eb may be formed, thereby combining the projected portions.

Hereinbelow, a description is given of a fluid control input device according to a fifth embodiment of the present invention.

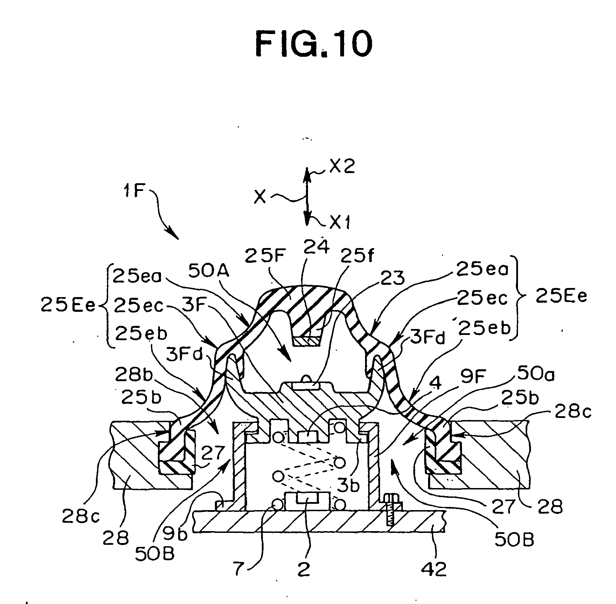

Fig. 10 is a sectional view showing the detailed structure of a fluid control input device (fluid control input member 1F) according to the fifth embodiment.

The structure according to the fifth embodiment basically uses the same structure according to the first embodiment. However, the structures of an operating button 3F, a button receiving member 9F for receiving the operating button 3F, and a rubber cover 25F for covering the components are different from those according to the first embodiment. Therefore, the same components according to the first embodiment are designated by the same reference numerals, a detailed description is omitted, and only different portions are hereinbelow described in detail. An endoscope using the fluid control input device according to the fifth embodiment and a fluid control apparatus connected to the endoscope are the same as those according to the first embodiment, therefore, are referred to Fig. 1, and a drawing and a description thereof are omitted. Incidentally, a description is given of the case of using an operating member (fluid control input device) for switching operation of on/off (start or end) of the absorbing operation and for adjusting operation of the amount of absorption according to the fifth embodiment.

The fluid control input device according to the fifth embodiment, the click mechanism including the click spring 6 and the projected portions 3a arranged according to the first embodiment is omitted, and the rubber cover 25F is bent and is folded two times, thereby generating the sense of click operation two times. With the sense of click operation when the rubber cover 25F is bent and is folded at the second time, the substantially same function as that of the click mechanism according to the first embodiment is obtained.

In accordance therewith, the button receiving member 9F according to the fifth embodiment has the flange unit 9b at the bottom end and is substantially cylindrically formed with the openings at both ends, similarly to the first embodiment. However, three groove notches (9a in Fig. 2) on the circumference are omitted.

The opening of the button receiving member 9F at the top end has a slightly smaller diameter dimension than that of outer diameter of a flange unit 3Fb, which will be described later, at the bottom end of the operating button 3F so as to lock the flange unit 3Fb of the operating button 3F. Therefore, the operating button 3F inserted in the button receiving member 9F from the bottom end thereof is not pulled-out upstream. The operating button 3F is always repulsed in the direction shown by an arrow X2 shown in Fig. 10 by the repulsive force of the repulsive spring 7.

The operating button 3F is a substantially cylindrical member having the flange unit 3Fb at the bottom end thereof. On the outer circumference of the operating button 3F on the side of the top end, a projected portion 3Fd for supporting a predetermined position (refer to reference numeral 25ec in Fig. 10) of the rubber cover 25F is formed, as will be described later.

A micro switch 23 is arranged at a predetermined position on the top-end surface of the operating button 3F, with a switch portion directed to the top. The micro switch 23 is a switch member with which a pressing member 24 arranged at the distal end of a projected portion 25Ff formed to the inner surface of the top of the rubber cover 25F, which will be described later, comes into contact, thereby switching the on/off operation of the absorbing operation.

Incidentally, similarly to the first embodiment, the light-emitting device 4 is arranged at the position facing the light receiving surface of the optical sensor 2 arranged onto the fixing substrate 42 on the bottom-end surface of the operating button 3F.

As mentioned above, the rubber cover 25F according to the fifth embodiment watertightly covers the entire components in the fluid control input member 1F, and has a function of watertight covering means or a covering member. Further, the rubber cover 25F is formed so that at least different two portions of the rubber cover 25F are bent and are folded by the rubber cover 25F itself and by the pressing operation from the operating button 3F at different timings.

As mentioned above, on the inner surface of the top of the rubber cover 25F, the projected portion 25Ff projected in the down direction is formed. The pressing member 24 is adhered and is fixed, by an adhesive or the like, to the distal end of the projected portion 25Ff. Further, the pressing member 24 contains a resin piece which comes into contact with the micro switch 23 when the rubber cover 25F is pressed in the direction shown by an arrow X1 in Fig. 10.

A smooth curved portion is formed at a portion ranging from the top to the side of the rubber cover 25F (portion 25Ee). The projected portion 3Fd of the operating button 3F is fixed to the inner surface corresponding to a portion 25ec in the middle of the curved portion 25Ee. Therefore, in the curved portion 25Ee of the rubber cover 25F, with the border of the middle portion of the portion 25ec, a first curved-portion 25ea is arranged to the top side, serving as a first shape bendable portion, and a second curved portion 25eb is arranged to the bottom side, serving as a second shape bendable portion.

In the rubber cover 25F, a first space 50A is formed in a space on the top side of the operating member 3F, and a second space 50B is formed, on the bottom side of the operating member 3F, in a space between the button receiving member 9F and the hole portion 28b of the operating unit 28. Incidentally, in this case, when the inner portion of the first space 50A is sealed, the rubber cover 25F is not folded in a predetermined form when the rubber cover 25F is pressed and is bent. Corresponding to this, for example, the operating button 3F needs a predetermined through-hole (not shown in Fig. 10) for the air coming and going between the first space 50A and the second space 50B.

Incidentally, similarly to the first embodiment, an attaching portion of the rubber cover 25F to the operating unit 28 is fixed by fitting and adhering the end 25b to a predetermined position (groove portion 28c) of the operating unit 28. The portion is shown simplified for the purpose of a brief drawing in Fig. 10.

The fluid control input member 1F according to the fifth embodiment has the above-mentioned two-step switch structure.

In the rubber cover 25F with the above structure, first, the first curved-portion 25ea is folded toward the first space 50A and is bent at a predetermined position in accordance with the pressing operation of the top of the rubber cover 25F. In the process of the bending, the change in amount of force is temporarily and sharply generated.

At the same timing for folding the first curved-portion 25ea or at the timing after/before the first curved-portion 25ea is folded, the pressing member 24 comes into contact with the switch portion of the micro switch 23 and presses it. Thus, the micro switch 23 is switched to the on-state or off-state, thereby generating a predetermined signal. The fluid control apparatus 11 receives an on-signal or off-signal from the micro switch 23, and performs predetermined control processing, thereby starting or stopping predetermined absorbing processing with the minimum amount of fluid.

Incidentally, at the timing at which the first curved-portion 25ea is folded and the micro switch 23 is switched, the amount of pressing force of the rubber cover 25F is reset. Then, the rubber cover 25F is reset to the normal state shown in Fig. 10 by its restoring force. In this case, the state switched by the micro switch 23 is kept.

Specifically, when the state before pressing the rubber cover 25F is in the stop state of the absorbing operation, the switching operation to the on-state using the micro switch 23 at this time starts the absorbing operation with the minimum amount of fluid. Therefore, even if the amount of pressing force of the rubber cover 25F is reset and the state of the rubber cover 25F is restored to the normal state, the absorbing operation with the minimum amount of fluid in this case is kept.

If the absorbing operation is being executed before pressing the rubber cover 25F, the switching operation to the off-state using the micro switch 23 at this time stops the absorbing operation. Therefore, when the amount of pressing force of the rubber cover 25F is reset and the state of the rubber cover 25F is restored to the normal state, the stop state of the absorbing operation does not change.

When the first curved-portion 25ea is folded and the rubber cover 25F is further pressed, the folding state of the first curved-portion 25ea is kept and the operating button 3F is simultaneously pressed. Then, the second curved-portion 25eb is folded in the second space 50B and is bent in a predetermined form. In the process of the bending, the change in amount of force is temporarily and sharply generated.

In this case, the simultaneous pressing operation of the operating button 3F enables the operating button 3F to start to move in the direction shown by the arrow X1 in Fig. 10. In accordance with the positional change, a predetermined signal is outputted from the optical sensor 2. The fluid control apparatus 11 receives the output signal and performs the adjusting and control processing of the amount of absorption.

That is, the amount of absorption is adjusted depending on the position of the operating button 3F. Therefore, the operator adjusts the amount of pressing force of the operating button 3F, thereby arbitrarily adjusting the amount of absorption.

At the same timing for temporarily and sharply generating the change in amount of force by folding the second curved-portion 25eb or the timing before/after temporarily and sharply generating the change in amount of force, in accordance with the output signal from the optical sensor 2, the fluid control apparatus 11 performs the control processing so that the amount of absorption is maximum.

Incidentally, with respect to the rubber cover 25F and the operating button 3F, the rubber cover 25F is reset to the state shown in Fig. 10 by the repulsive force of the repulsive spring 7 in the direction shown by the arrow X2 in Fig. 10 and the restoring force of the rubber cover 25F. Therefore, only the release of the amount of pressing force of the rubber cover 25F and the operating button 3F by the operator adjusts the absorbing amount of fluid in the releasing direction of the absorbing amount of fluid. When the amount of pressing force is completely reset, the rubber cover 25F is restored to the normal state shown in Fig. 10. In this case, the shape restoring of the rubber cover 25F does not switch the on/off operation of the micro switch 23, thereby keeping the absorbing operation with the minimum amount of absorption.

That is, unless the rubber cover 25F is pressed again and the micro switch 23 switches the on/off operation, the absorbing operation in progress of execution does not shift to the stop state.

As mentioned above, according to the fifth embodiment, the same advantages according to the first embodiment are obtained. Further, the click mechanism using the click spring and the like according to the first embodiment is omitted, and the rubber cover 25F is bent at two steps and is folded, thereby generating the sense of click operation two times. Substantially the same functions of click mechanism according to the first embodiment are obtained. Therefore, the omission of components realizes the simple mechanism and this contributes to the reduction in manufacturing costs.

The operating member (fluid control input device) is used to switch the on/off operation (start or end) of the absorbing operation and to adjust the amount of absorption. Thus, the amount of absorption is easily adjusted. When the amount of absorption is not adjusted and the absorption is always executed with the maximum amount of absorption, there is a problem that unnecessary absorbing force is generated and the adsorption to the wall of body cavity happens. However, the amount of absorption is adjusted, thereby easily solving the above problem.

Incidentally, the present invention is not limited to the above embodiments and can be modified without departing from the essentials of the present invention.

### Industrial Applicability

According to the present invention, as mentioned above, the fluid control input device for endoscope according to the present invention is preferably applied to a fluid control apparatus for an endoscope, which supplies the fluid and absorbs the intracavital tissue via a channel arranged in the endoscope.

## Claims

1. A fluid control input device (1, 11) for endoscope comprising:
moving means (3) that adjusts the amount of flow of the fluid supplied to a channel (16, 17)in the endoscope and switches the type of fluid by using an amount of pressing force;
position restoring means (7) that restores the moving means (3) to be always at a predetermined position;
signal generating means (2, 4) that generates a predetermined signal depending on the positional change in accordance with the amount of pressing force of the moving means (3);
watertightly covering means (25) that watertightly covers the entire components including the moving means; and
amount-of-force changing means (6) that temporarily and sharply generates the change in amount of force, at the same timing of a predetermined signal generated depending on the positional change of the moving means or the change in amount of restoring force of the position restoring means or a predetermined timing before/after generating the signal,
wherein notifying means sends a notification that a predetermined signal is generated by the signal generating means, to an operator by using the change in amount of force generated by the amount-of-force changing means.

2. A fluid control input device for endoscope according to Claim 1, wherein the watertightly covering means is bent in accordance with the pressing operation of the moving means, and is formed to be folded in the direction in substantially parallel with the moving direction of the moving member, and
a space in which the watertightly covering means is bent and is folded is formed between the moving means and the watertightly covering means.

3. A fluid control input device for endoscope according to Claim 1, wherein the change in amount of force that is temporarily and sharply generated by the amount-of-force changing means is set in the direction for reducing the amount of force.

4. A fluid control input device for endoscope according to Claim 2, wherein the change in amount of force that is temporarily and sharply generated by the amount-of-force changing means is set in the direction for reducing the amount of force.

5. A fluid control input device for endoscope according to Claim 1, wherein the watertightly covering means contains an elastically modifying material.

6. A fluid control input device for endoscope according to Claim 1, wherein two or more amount-of-force changing means are arranged.

## Patentansprüche

1. Eingabeeinrichtung (1, 11) zur Fluidsteuerung bei einem Endoskop, folgendes aufweisend:
eine bewegliche Einrichtung (3), die eine einem Kanal (16, 17) in einem Endoskop zugeführte Strömung des Fluids einstellt und die Art des Fluids mittels Druckkraft einstellt;
eine Rückstelleinrichtung (7), welche die bewegliche Einrichtung (3) rückstellt, sodass sie in einer vorgegebenen Stellung ist;
eine Signal-Erzeugungseinrichtung (2, 4), die ein vorgegebenes Signal in Abhängigkeit einer Positionsänderung entsprechend der Größe der Druckkraft der beweglichen Einrichtung (3) erzeugt;
eine wasserdichte Abdeckung (25), die die Komponenten einschließlich der beweglichen Einrichtung wasserdicht abdeckt; und
eine Kraftstärkenwechseleinrichtung (6), die zeitweise und abrupt den Kraftwechsel erzeugt, gleichzeitig zu einem vorgegebenen Signal, welches in Abhängigkeit von einer Positionsänderung der beweglichen Einrichtung oder einer Änderung in der Rückstellkraft der Rückstelleinrichtung oder einer vorgegebenen Zeitspanne vor/nach der Erzeugung des Signals erzeugt wird,
wobei eine Unterrichtungseinrichtung eine Unterrichtung, dass ein vorgegebenes Signal durch die Signalerzeugungseinrichtung erzeugt ist, an eine Bedienungsperson unter Verwendung der Änderung der von der Krafterzeugungseinrichtung erzeugten Kraftstärke sendet.

2. Eingabeeinrichtung zur Fluidsteuerung bei einem Endoskop gemäß Anspruch 1, wobei die Einrichtung zur wasserdichten Abdeckung entsprechend einer Druckbetätigung der beweglichen Einrichtung gebogen wird und so geformt ist, dass sie in einer Richtung faltbar ist, die im Wesentlichen parallel zur Bewegungsrichtung des beweglichen Teils ist, und wobei
ein Raum, in dem die wasserdichte Abdeckung gebogen und gefaltet wird, zwischen der beweglichen Einrichtung und der wasserdichten Abdeckung ausgeformt ist.

3. Eingabeeinrichtung zur Fluidsteuerung bei einem Endoskop gemäß Anspruch 1, wobei die Kraftstärkenänderung, die zeitweise und abrupt durch die Kraftstärkenänderungseinrichtung erzeugt wird, in Richtung zur Reduzierung der Kraftstärke eingestellt ist.

4. Eingabeeinrichtung zur Fluidsteuerung bei einem Endoskop gemäß Anspruch 2, wobei die Kraftstärkenänderung, die zeitweise und abrupt durch die Kraftstärkenänderungseinrichtung erzeugt wird, in Richtung zur Reduzierung der Kraftstärke eingestellt ist.

5. Eingabeeinrichtung zur Fluidsteuerung bei einem Endoskop gemäß Anspruch 1, wobei die wasserdichte Abdeckung ein elastisch modifiziertes Material aufweist.

6. Eingabeeinrichtung zur Fluidsteuerung bei einem Endoskop gemäß Anspruch 1, wobei zwei oder mehr Kraftstärkenänderungseinrichtungen vorgesehen sind.

## Revendications

1. Dispositif d'entrée de commande de fluide (1, 11) pour endoscope comprenant :
un élément mobile (3) qui ajuste la quantité de flux du fluide alimenté vers un canal (16, 17) dans l'endoscope et commute le type de fluide en utilisant une quantité de force de pressage ;
un moyen de restauration de position (7) qui restaure l'élément mobile (3) pour qu'il soit toujours à une position prédéterminée ;
un moyen générant un signal (2, 4) qui génère un signal prédéterminé en fonction du changement de position selon la quantité de force de pressage de l'élément mobile (3) ;
un moyen de recouvrement étanche à l'eau (25) qui recouvre de façon étanche à l'eau les composants entiers y compris l'élément mobile ; et
un moyen de changement de quantité de force (6) qui génère temporairement et brusquement le changement dans la quantité de force, au même moment qu'un signal prédéterminé généré en fonction du changement de position de l'élément mobile ou du changement dans la quantité de force de restauration du moyen de restauration de position ou à un moment prédéterminé avant/après la génération du signal,
dans lequel un moyen de notification envoie une notification qu'un signal prédéterminé est généré par le moyen de génération de signal, à un opérateur en utilisant le changement dans la quantité de force générée par le moyen de changement de quantité de force.

2. Dispositif d'entrée de commande de fluide pour un endoscope selon la revendication 1, dans lequel le moyen de recouvrement étanche à l'eau est courbé selon l'opération de pressage de l'élément mobile, et est formé pour être plié dans la direction sensiblement parallèle à la direction de déplacement de l'élément mobile, et
un espace dans lequel le moyen de recouvrement étanche à l'eau est courbé et est plié, est formé entre l'élément mobile et le moyen de recouvrement étanche à l'eau.

3. Dispositif d'entrée de commande de fluide pour un endoscope selon la revendication 1, dans lequel le changement dans la quantité de force qui est générée temporairement et brusquement par le moyen de changement de quantité de force, est placé dans la direction pour réduire la quantité de force.

4. Dispositif d'entrée de commande de fluide pour un endoscope selon la revendication 2, dans lequel le changement dans la quantité de force qui est générée temporairement et brusquement par le moyen de changement de quantité de force, est placé dans la direction pour réduire la quantité de force.

5. Dispositif d'entrée de commande de fluide pour un endoscope selon la revendication 1, dans lequel le moyen de recouvrement étanche à l'eau contient un matériau de modification élastique.

6. Dispositif d'entrée de commande de fluide pour un endoscope selon la revendication 1, dans lequel deux moyens de changement de quantité de force ou plus sont agencés.
